# EUROPEAN PATENT SPECIFICATION

(11) **EP 2 632 407 B1**
(45) Date of publication and mention of the grant of the patent: **23.08.2017**
(21) Application number: 11749596.0
(22) Date of filing: 15.07.2011
(51) Int. Cl.: A61F 13/00, A61M 1/00, A61F 13/02

(54) **INTERACTIVE, WIRELESS REDUCED-PRESSURE DRESSINGS, METHODS, AND SYSTEMS**
INTERAKTIVE DRAHTLOSE UNTERDRUCKWUNDVERBÄNDE, VERFAHREN UND SYSTEME
PANSEMENTS À PRESSION RÉDUITE SANS FILS, INTERACTIFS, PROCÉDÉS ET SYSTÈMES ASSOCIÉS

(30) Priority: 01.12.2010 US 418730 P; 27.10.2010 US 407194 P; 22.02.2011 US 201161445383 P; 22.02.2011 US 201161445338 P
(43) Date of publication of application: 04.09.2013
(73) Proprietor: KCI Licensing, Inc., San Antonio, TX 78265 (US)
(72) Inventor: LOCKE, Christopher, Brian, Bournemouth Dorset BH9 35D (GB); COULTHARD, Richard, Daniel John, Verwood Dorset BH31 6LL (GB)
(74) Representative: Cordina, Kevin John
(86) International application number: PCT/US2011/044189
(87) International publication number: WO 2012/057882

(56) References cited:
- WO-A1-2009/093116
- WO-A2-2009/089390
- DE-U1- 20 213 196
- KR-A- 20090 092 548

## Description

### RELATED APPLICATION

The present invention claims the benefit, under 35 USC § 119(e), of the filing of U.S. Provisional Patent Application serial number 61/407,194, entitled "System and Methods For Electrically Detecting The Presence of Exudate In Reduced-Pressure Dressings," filed 27 October 2010, which is incorporated herein by reference for all purposes [VAC.0975PRO1]; U.S. Provisional Patent Application serial number 61/418,730, entitled "Systems and Methods for Electrically Detecting the Presence of Exudate in Dressings," filed 1 December 2010, which is incorporated herein by reference for all purposes [VAC.0975PRO2]; U.S. Provisional Patent Application serial number 61/445,383, entitled "Interactive, Wireless Reduced-Pressure Dressings, Methods, and Systems," filed 22 February 2011, which is incorporated herein by reference for all purposes [VAC.0999PRO]; and U.S. Provisional Patent Application serial number 61/445,338, entitled "Reduced-Pressure Systems, Dressings, and Methods Employing a Wireless Pump," filed 22 February 2011, which is incorporated herein by reference for all purposes [VAC.1000PRO].

### FIELD

The present disclosure relates generally to medical treatment systems and, more particularly, but not by way of limitation, to interactive, wireless dressings, methods, and systems for use with reduced pressure.

### BACKGROUND

Clinical studies and practice have shown that providing a reduced pressure in proximity to a tissue site augments and accelerates the growth of new tissue at the tissue site. The applications of this phenomenon are numerous, but application of reduced pressure has been particularly successful in treating wounds. This treatment (frequently referred to in the medical community as "negative pressure wound therapy," "reduced pressure therapy," or "vacuum therapy") provides a number of benefits, which may include faster healing and increased formulation of granulation tissue. Typically, when applied to open wounds, reduced pressure is applied to tissue through a porous pad or other manifold device. The porous pad distributes reduced pressure to the tissue and channels fluids that are drawn from the tissue. At times, a patient may have a large wound requiring treatment at numerous sites or has a plurality of tissue sites requiring treatment. At times, reduced pressure may also be used within a body cavity to remove fluids among other things.

WO 2009/089390 A2 discloses a system for providing reduced or negative pressure to treat a wound.

WO 2009/093116 A1 discloses a wound treatment system with a wound dressing suitable for the application of a negative pressure to a wound, with a sensor associated with the wound.

### SUMMARY

According to an illustrative embodiment, a system for treating at least one tissue site with reduced pressure includes a first, wireless, reduced-pressure dressing for disposing proximate to the tissue site. The first, wireless, reduced-pressure dressing includes a distribution manifold, a drape covering at least a portion of the distribution manifold, a Radio Frequency Identification (RFID) antenna, a first processor coupled to the RFID antenna, and a first sensor coupled to the first processor. The system also includes a remote base unit and a reduced-pressure source. The reduced-pressure source is fluidly coupled to the distribution manifold. The remote base unit includes a RFID antenna and a second processor.

According to another illustrative embodiment, a wireless, reduced-pressure dressing for treating a tissue site with reduced pressure includes a distribution manifold for placing adjacent to the tissue site and for providing reduced pressure to the tissue site, a drape covering at least a portion of the distribution manifold, a first processor, a RFID antenna coupled to the first processor, and a first sensor coupled to the first processor. The first sensor may be a pressure sensor.

According to another illustrative embodiment, a method for treating at least one tissue site with reduced pressure includes the steps of disposing a first, wireless, reduced- pressure dressing proximate to a first tissue site. The first, wireless, reduced-pressure dressing includes a distribution manifold for placing adjacent to the tissue site and for providing reduced pressure to the tissue site, a drape covering at least a portion of the distribution manifold, a first processor, a RFID antenna coupled to the first processor, and a first sensor coupled to the first processor. The first sensor is a pressure sensor. The method further includes providing a remote base unit that has a RFID reader, providing reduced pressure to the distribution manifold, transmitting a pressure inquiry signal from the RFID reader to the first, wireless, reduced-pressure dressing, and receiving a pressure message signal from the first, wireless, reduced-pressure dressing.

According to another illustrative embodiment, a method for treating at least one tissue site with reduced pressure includes the steps of disposing a first, wireless, reduced-pressure dressing proximate to a first tissue site. The first, wireless, reduced-pressure dressing includes a distribution manifold for placing adjacent to the tissue site and for providing reduced pressure to the tissue site, a drape covering at least a portion of the distribution manifold, a first processor, a RFID antenna coupled to the first processor, and a first sensor coupled to the first processor. The first sensor may be a pressure sensor. The method further includes providing a remote base unit having a RFID reader. The remote base unit also includes a second processor coupled to the RFID reader. The method further includes providing a reduced-pressure source; transmitting an ID inquiry signal from the remote base unit to the first, wireless, reduced-pressure dressing; receiving the ID inquiry signal at the first, wireless, reduced-pressure dressing and producing an ID message signal; transmitting the ID message signal from the first, wireless, reduced-pressure dressing to the remote base unit; receiving the ID message signal at the remote base unit; determining if the ID message signal is on an approved list; and activating the reduced-pressure source to provide reduced pressure to the distribution manifold if the ID message signal represents a dressing that is on the approved list or indicating an error if the ID message signal represents a dressing that is not on the approved list.

According to another illustrative embodiment, a system for treating at least one tissue site with reduced pressure includes a first, wireless, reduced-pressure dressing for disposing proximate to the tissue site. The first, wireless, reduced-pressure dressing includes a distribution manifold, a drape covering at least a portion of the distribution manifold, a reduced-pressure interface for providing reduced pressure to the distribution manifold, a first RFID antenna, a first processor coupled to the first RFID antenna, a first sensor coupled to the first processor, and a membrane. The membrane covers at least a portion of the reduced-pressure interface and is initially in an occlusive state that prevents or hinders fluid flow through the reduced-pressure interface. The first, wireless, reduced-pressure dressing further includes a dissolution element proximate to the membrane that is adapted to change the membrane from the occlusive state to a non-occlusive state. When changed to the non-occlusive state, the membrane allows flow through reduced-pressure interface. The system further includes a remote base unit having a RFID reader and a second processor. The system also includes a reduced-pressure source fluidly coupled to the distribution manifold. The second processor is configured to transmit an activation signal with the RFID reader to the first, wireless, reduced-pressure dressing and wherein in response to the activation signal, the first processor is configured to deliver power to the dissolution element to change the membrane from the occlusive state to the non-occlusive state. The second processor is configured to transmit an ID inquiry signal with the RFID reader to the first, wireless, reduced-pressure dressing, and wherein, in response to the ID inquiry signal, the first processor is configured to transmit an ID message signal with the first RFID antenna. The second processor is adapted to receive the ID message signal via the RFID reader and to determine if the ID message signal represents an acceptable dressing.

According to another illustrative embodiment, a system for treating a tissue site with reduced pressure includes a wireless, reduced-pressure dressing that includes a distribution manifold, a drape covering the distribution manifold, and a reduced-pressure interface. The system also includes a WISP device associated with the wireless, reduced-pressure dressing, a remote base unit comprising a RFID reader, and a reduced-pressure source fluidly coupled to the wireless, reduced-pressure dressing.

According to another illustrative embodiment, a method for treating at least one tissue site with reduced pressure includes disposing a first, wireless, reduced-pressure dressing proximate to a first tissue site. The first, wireless, reduced-pressure dressing includes a distribution manifold for placing adjacent to the tissue site and for providing reduced pressure to the tissue site, a drape covering at least a portion of the distribution manifold, a reduced-pressure interface, and a membrane. The membrane is initially occlusive and covers at least a portion of the reduced-pressure interface. The membrane is adapted to prevent fluid flow through the reduced-pressure interface when in the occlusive state. The first, wireless, reduced-pressure dressing also includes a first processor, a RFID antenna coupled to the first processor, a first sensor coupled to the first processor, and a dissolution element proximate to the membrane that is operable, when activated, to change the membrane from an occlusive state to a non-occlusive state. The first sensor is a pressure sensor. The dissolution element is coupled to the first processor. The method further includes providing a remote base unit having a RFID reader. The remote base unit includes a second processor. The method also involves providing a reduced-pressure source fluidly coupled to the reduced-pressure interface, transmitting an activation signal from the remote base unit to the first, wireless, reduced-pressure dressing, whereupon the first processor activates the dissolution element to change the membrane to the non-occlusive state, and providing reduced pressure from the reduced-pressure source to the reduced-pressure interface.

Other features and advantages of the illustrative embodiments will become apparent with reference to the drawings and detailed description that follow.

### BRIEF DESCRIPTION OF THE DRAWINGS

FIGURE 1 is a schematic diagram, with a portion shown in cross section, of an illustrative embodiment of a system for treating at least one tissue site with reduced pressure;
FIGURE 2 is a schematic top view of a first, wireless, reduced-pressure dressing of the system of FIGURE 1;
FIGURE 3 is a schematic diagram, with a portion shown in cross section, of an illustrative embodiment of a system for treating at least one tissue site with reduced pressure;
FIGURE 4 is a schematic top view of an illustrative embodiment of a first, wireless, reduced-pressure dressing;
FIGURE 5 is a schematic diagram, with a portion shown in cross section, of an illustrative embodiment of a system for treating at least one tissue site with reduced pressure; and
FIGURES 6A and 6B are an illustrative embodiment of a process flow chart for a system for treating at least one tissue site with reduced pressure.

### DETAILED DESCRIPTION OF ILLUSTRATIVE EMBODIMENTS

In the following detailed description of the illustrative embodiments, reference is made to the accompanying drawings that form a part hereof. These illustrative embodiments are described in sufficient detail to enable those skilled in the art to practice the invention, and it is understood that other embodiments may be utilized and that logical structural, mechanical, electrical, and chemical changes may be made without departing from the spirit or scope of the invention. To avoid detail not necessary to enable those skilled in the art to practice the embodiments described herein, the description may omit certain information known to those skilled in the art. The following detailed description is not to be taken in a limiting sense, and the scope of the illustrative embodiments are defined only by the appended claims.

In treating a tissue site or sites on a patient with reduced pressure, it is typically desirable to maintain the reduced pressure in a therapeutic range. While the amount and nature of reduced pressure applied to a tissue site will typically vary according to the application, the reduced pressure will typically be between -5 mm Hg and -500 mm Hg and more typically -75 mm Hg and -300 mm Hg, and more typically still, -100 to -200 mm Hg. In some instances, failure to provide reduced pressure to a tissue site can lead to serious consequences. Accordingly, it may be desirable to monitor the pressure at each tissue site undergoing treatment.

To accommodate multiple tissue sites, e.g., multiple wounds, multiple conduits may be used to deliver reduced pressure. For example, a single reduced-pressure source may be used with the multiple conduits branched off of one conduit. Currently, pressure monitoring is typically located in existing reduced-pressure sources and only one conduit communicates pressure to the reduced-pressure source. If only one conduit, which is associated with one tissue site, is monitored, as is the case when monitoring is done at the reduced-pressure source alone, pressures at other tissue sites are unmonitored. With at least some of the illustrative embodiments herein, each tissue site of the plurality of tissue sites is monitored so that issues with reduced pressure delivery may be identified and addressed. Moreover, other parameters may be monitored to track progress or identify issues with healing of the tissue sites.

Reduced pressure may be used to treat open wounds to promote the granulation tissue. Reduced pressure may also be applied to a tissue site internal to a patient to remove fluids. For example, reduced pressure may be used to remove ascites from a patient's abdomen. In such situations it may be desirable to know the pressure at the internal location as well as other parameters. Reduced pressure may be used for other applications to promote healing. The illustrative embodiments herein may be operable to perform these tasks.

The illustrative embodiments herein involve using Radio Frequency Identification (RFID) technology, including enhanced RFID technology, to wirelessly transmit and receive sensing information from a reduced-pressure dressing. RFID uses a RFID tag or label that is on a target and a RFID reader that energizes and reads a signal from the RFID tag. Most RFID tags include an integrated circuit for storing and processing information, a modulator, and demodulator. To enhance the RFID tag, a microcontroller (or processor) and sensor are incorporated that allow sensing and optional computational functions to occur. RFID tags can be passive tags, active RFID tags, and battery-assisted passive tags. Generally, passive tags use no battery and do not transmit information unless they are energized by a RFID reader. Active tags have an on-board battery and can transmit autonomously (i.e., without being energized by a RFID reader). Battery-assisted passive tags typically have a small battery on-board that is activated in the presence of a RFID reader.

In one illustrative embodiment, the enhanced RFID technology is a Wireless Identification and Sensing Platform (WISP) device. WISPs involve powering and reading a WISP device, analogous to a RFID tag (or label), with a RFID reader. The WISP device harvests the power from the RFID reader's emitted radio signals and performs sensing functions (and optionally performs computational functions). The WISP device transmits a radio signal with information to the RFID reader. The WISP device receives power from the RFID reader. The WISP device has a tag or antenna that harvests energy and a microcontroller (or processor) that can perform a variety of tasks, such as sampling sensors. The WISP device reports data to the RFID reader. In one illustrative embodiment, the WISP device includes an integrated circuit with power harvesting circuitry, demodulator, modulator, microcontroller, sensors, and may include one or more capacitors for storing energy. A form of WISP technology has been developed by Intel Research Seattle (www.seattle.intel-research.net/wisp/).

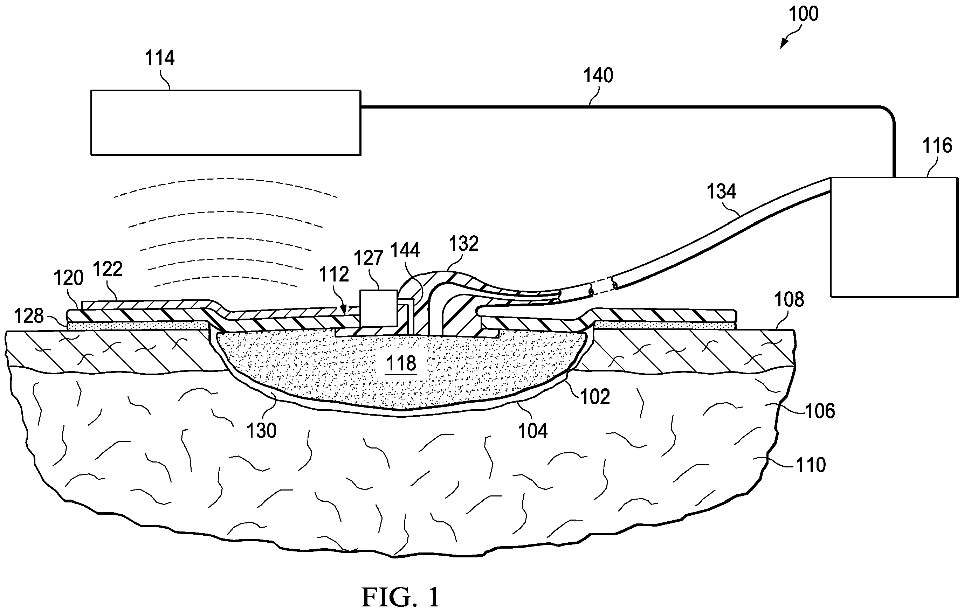

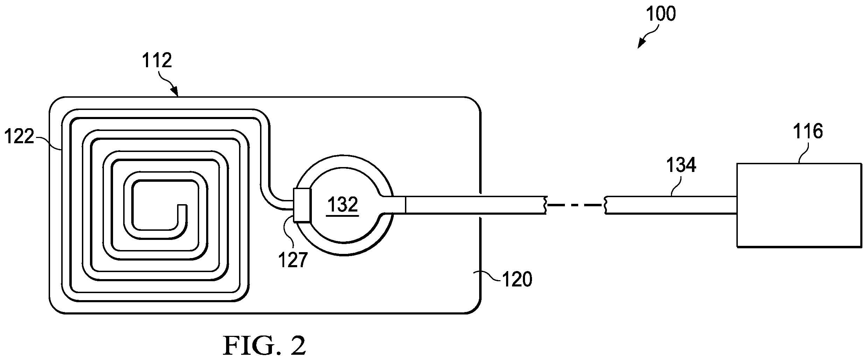

Referring now to the drawings and initially to FIGURES 1-3, a system 100 for treating at least one tissue site 102, e.g., a wound site 104, with reduced pressure is presented. The illustrative wound site 104 is shown through epidermis 108 and into the subcutaneous tissue 110 of a patient 106. The tissue site 102 may be the bodily tissue of any human, animal, or other organism, including bone tissue, adipose tissue, muscle tissue, dermal tissue, vascular tissue, connective tissue, cartilage, tendons, ligaments, or any other tissue. Treatment of tissue site 102 may include removal of fluids, e.g., exudate or ascites. Unless otherwise indicated, as used throughout this document, "or" does not require mutual exclusivity. Treatment of a tissue site 102 may include removal of fluids, e.g., exudate or ascites.

The system 100 may be used to treat the tissue site 102 with reduced pressure to help form granulation tissue (reduced pressure therapy), to remove fluids with out promoting tissue growth, or any other purpose for which reduced pressure is helpful. The system 100 includes a first, wireless, reduced-pressure dressing 112 for disposing proximate to the tissue site 102, a remote base unit 114, and a reduced-pressure source 116.

The first, wireless, reduced-pressure dressing 112 may include a distribution manifold 118, a sealing member 120 (or drape) covering at least a portion of the distribution manifold 118, a first RFID antenna 122, a first processor 124 coupled to the first RFID antenna 122, and a first sensor 126 coupled to the first processor 124. The first processor 124 and first sensor 126 may be associated with a board 127 or housing.

The sealing member 120 creates a fluid seal over the distribution manifold 118 on a portion of a patient's epidermis 108 or may help provide a fluid seal at other locations. "Fluid seal," or "seal," means a seal adequate to maintain reduced pressure at a desired tissue site given the particular reduced-pressure source or subsystem involved. The sealing member 120, may include an attachment device 128. The sealing member 120 creates a sealed space 130 in which the distribution manifold 118 may be positioned. A reduced-pressure interface 132 may be placed through an aperture in the sealing member 120 to provide reduced pressure into the sealed space 130 and in particular to the distribution manifold 118. Other wireless reduced-pressure dressings may be used as part of the system 100 to accommodate multiple tissue sites, and the additional wireless reduced-pressure dressings may be analogous to the first, wireless, reduced-pressure dressing 112.

With respect to the distribution manifold 118, the term "manifold" as used herein generally refers to a substance or structure that is provided to assist in applying reduced pressure to, delivering fluids to, or removing fluids from a tissue site, e.g., tissue site 102. The distribution manifold 118 typically includes a plurality of flow channels or pathways that distribute fluids provided to and removed from the tissue site 102 around the distribution manifold 118. In one illustrative embodiment, the flow channels or pathways are interconnected to improve distribution of fluids provided or removed from the tissue site 102. The distribution manifold 118 may also be a biocompatible material that is capable of being placed in contact with the tissue site 102 and distributing reduced pressure to the tissue site 102. Examples of manifolds 118 may include, without limitation, devices that have structural elements arranged to form flow channels, such as, for example, cellular foam, open-cell foam, porous tissue collections, liquids, gels, and foams that include, or cure to include, flow channels. The examples are not mutually exclusive.

The distribution manifold 118 may be porous and may be made from foam, gauze, felted mat, or any other material suited to a particular biological application. In one embodiment, the distribution manifold 118 is a porous foam and includes a plurality of interconnected cells or pores that act as flow channels. The porous foam may be a polyurethane, open-cell, reticulated foam such as GranuFoam® material manufactured by Kinetic Concepts, Incorporated of San Antonio, Texas. Other embodiments may include "closed cells." In some situations, the distribution manifold 118 may also be used to distribute fluids such as medications, antibacterials, growth factors, and various solutions to the tissue site 102. Other layers may be included in or on the distribution manifold 118 such as absorptive materials, wicking materials, hydrophobic materials, and hydrophilic materials.

In one illustrative embodiment, the distribution manifold 118, or portions of the distribution manifold 118, may be constructed from bioresorbable materials that may remain in a patient's body following use of the wireless, reduced-pressure dressing 112. Suitable bioresorbable materials may include, without limitation, a polymeric blend of polylactic acid (PLA) and polyglycolic acid (PGA). The polymeric blend may also include without limitation polycarbonates, polyfumarates, and capralactones. The distribution manifold 118 may further serve as a scaffold for new cell growth, or a scaffold material may be used in conjunction with the distribution manifold 118 to promote cell growth. A scaffold is a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

The sealing member 120 may be any material that provides a fluid seal. The sealing member 120 may, for example, be an impermeable or semi-permeable, elastomeric material. "Elastomeric" means having the properties of an elastomer. It generally refers to a polymeric material that has rubber-like properties. More specifically, most elastomers have ultimate elongations greater than 100% and a significant amount of resilience. The resilience of a material refers to the material's ability to recover from an elastic deformation. Examples of elastomers may include, but are not limited to, natural rubbers, polyisoprene, styrene butadiene rubber, chloroprene rubber, polybutadiene, nitrile rubber, butyl rubber, ethylene propylene rubber, ethylene propylene diene monomer, chlorosulfonated polyethylene, polysulfide rubber, polyurethane (PU), EVA film, co-polyester, and silicones. Additional, specific examples of sealing member materials include a silicone drape, 3M Tegaderm® drape, polyurethane (PU) drape such as one available from Avery Dennison Corporation of Pasadena, California.

The attachment device 128 may be used to maintain the sealing member 120 against the patient's epidermis 108 or another layer, such as a gasket or additional sealing member, or another location. The attachment device 128 may take numerous forms. For example, the attachment device 128 may be a medically acceptable, pressure-sensitive adhesive that extends about a periphery, a portion, or the entire sealing member 120.

The first, wireless, reduced-pressure dressing 112 provides reduced pressure to one or more tissue sites 102. As used herein, "reduced pressure" generally refers to a pressure less than the ambient pressure at a tissue site that is being subjected to treatment. In most cases, this reduced pressure will be less than the atmospheric pressure at which the patient is located. Alternatively, the reduced pressure may be less than a hydrostatic pressure at the tissue site. Reduced pressure may initially generate fluid flow in the distribution manifold 118 and proximate the tissue site 102. As the hydrostatic pressure around the tissue site 102 approaches the desired reduced pressure, the flow may subside, and the reduced pressure may be maintained. Unless otherwise indicated, values of pressure stated herein are gauge pressures. Absolute pressure is referenced at times. The reduced pressure delivered may be constant or varied (patterned or random) and may be delivered continuously or intermittently. Although the terms "vacuum" and "negative pressure" may be used to describe the pressure applied to the tissue site, the actual pressure applied to the tissue site may be more than the pressure normally associated with a complete vacuum. Consistent with the use herein, an increase in reduced pressure or vacuum pressure typically refers to a relative reduction in absolute pressure.

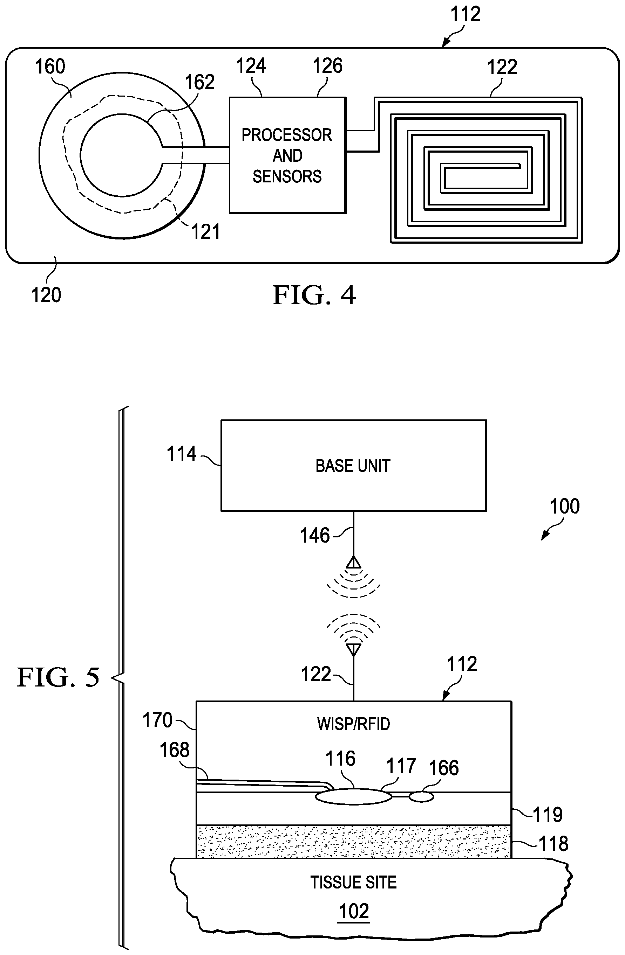

The reduced-pressure interface 132 may be a structure or means for fluidly coupling a reduced-pressure delivery conduit 134 to the distribution manifold 118. The reduced-pressure interface 132 may be a molded structure, a medical conduit (e.g., a portion of reduced-pressure delivery conduit 134) applied to the distribution manifold 118, or other device for coupling. As discussed in connection with FIGURE 4, a membrane, when in an occlusive state, may prevent or hinder flow of fluid through the reduced-pressure interface 132.

The reduced-pressure source 116 provides reduced pressure. The reduced-pressure source 116 may be any device or source for supplying a reduced pressure, such as a vacuum pump 136, wall suction, micro-pump, or other source. As an aspect of the reduced-pressure source 116 or as a separate member, a canister 138 may be included to receive and retain fluids 139. The remote base unit 114 may be electrically coupled by coupling 140, e.g., wire or wireless signal, to the reduced-pressure source 116 to provide a pressure control signal to control the delivery of reduced pressure to the first, wireless, reduced-pressure dressing 112 as will be discussed further below. The reduced-pressure source 116 and the remote base unit 114 may be an integrated unit in some embodiments. "Remote" as used in the context of "remote base unit" typically means displaced from a wireless, reduced-pressure dressing by a distance greater than several millimeters and may include a base unit immediately adjacent to a wireless, reduced-pressure dressing but not electrically coupled.

The first sensor 126 may be a pressure sensor and may be coupled to the first processor 124. The first processor 124 is coupled to, or in communication with, the first RFID antenna 122. The first sensor 126 may thus develop a signal indicative of pressure experienced at a desired site in or on the first, wireless, reduced-pressure dressing 112 and that signal may be referred to as a pressure message signal. In other embodiments, the first sensor 126 may be another type of sensor. For example, the first sensor may be one of the following: a pressure sensor, temperature sensor, pH sensor, humidity sensor, Volatile Organic Compounds (VOC) sensor, blood sensor, or growth factors sensor. The reduced-pressure interface 132 may include one or more pressure conduits or channels 144 to provide reduced pressure for sampling purposes to the first sensor 126.

A second sensor 142 may also be included and coupled to the first processor 124. The second sensor 142 may be any type of sensor such as those previously mentioned for the first sensor 126. It will be appreciated that the first RFID antenna 122, first processor 124, and first sensor 126 may comprise a Wireless Identification and Sensing Platform (WISP) device. The first sensor 126, second sensor 142, or any other sensors may be located at any location in the first, wireless, reduced-pressure dressing 112. In one illustrative embodiment, the first sensor 126 may be adjacent to the distribution manifold 118. In another illustrative embodiment, the first sensor 126 may be at a remote portion of the sealing member 120. In one illustrative embodiment, the sensors, e.g., the first sensor 126, may be laminated to the sealing member 120 or otherwise attached to a portion of the sealing member 120 and may sample pressure in the sealed space 130. While two sensors 126, 142 have been mentioned, it should be understood that additional sensors could be provided if desired.

In one illustrative embodiment, a battery is not associated with the first, wireless, reduced-pressure dressing 112. In other words, the first, wireless, reduced-pressure dressing 112 is passive. In such an embodiment, all the necessary energy for the first processor 124 and first sensor 126 (and any additional, optional sensors, e.g., second sensor 142) is harvested from signals received by the first RFID antenna 122 from the remote base unit 114. In other embodiments, a battery may be included to provide the necessary power. With the power from the battery, the first, wireless, reduced-pressure dressing 112 may transmit signals independent of any signal from the remote base unit 114. In another illustrative embodiment, a battery may be included to provide a portion of the necessary power.

The remote base unit 114 may include a board or other structure 115 that includes RFID reader 146 and a second processor 148. The remote base unit 114 may also include a first display 152, e.g., a pressure indicator 154. The first display 152 or indicator is for displaying a quantity measured by the first sensor 126 or an indication of adequate pressure. Similarly, additional displays or indicators may be included, e.g., a temperature display 156. One display may be used for displaying multiple items, e.g., the data from multiple sensors. A control panel 158, such as a push-button panel or graphical user interface, may be included to receive input from a user. The second processor 148 may be coupled by coupling 140 to the reduced-pressure source 116 to provide a control signal. The control signal allows for automated adjustments to the pressure at the tissue site 102 by controlling the pressure supplied by the reduced-pressure source 116.

The RFID reader 146 may be a transceiver for transmitting to and receiving signals from the first RFID antenna 122. If not already converted, the RFID reader 146 may convert received signals to digital format and provide the signals to the second processor 148. The remote base unit 114 may read signals as close as several millimeters away or as far as 30 feet or more away or any distance between, e.g., 5, 10, or 20 feet. The remote base unit 114 may poll as often as desired, e.g., every ½ second, every second, every hour, or any other time interval.

The second processor 148 includes memory and instructions necessary to perform various desired steps. For example, a pairing protocol may be executed. In the pairing protocol, the second processor 148 with the RFID reader 146 transmits an ID inquiry signal to the first, wireless, reduced-pressure dressing 112 to inquire about the identification of the first, wireless, reduced-pressure dressing 112. In response, the first processor 124 transmits an ID message signal. If the first, wireless, reduced-pressure dressing 112 is not suitable for use with the remote base unit 114 as determined by comparing the ID message with an approved list or by information contained in the ID message signal itself, the remote base unit 114 will not proceed with executing instructions as part of therapy. This protocol should safeguard against using a dressing that is not approved and which may underperform with the remote base unit 114.

As an illustrative example, the second processor 148, RFID reader 146, first RFID antenna 122, and first processor 124 may be configured to perform the following steps: transmitting an ID inquiry signal from the remote base unit 114 to the first, wireless, reduced-pressure dressing 112; receiving the ID inquiry signal at the first, wireless, reduced-pressure dressing 112 and producing an ID message signal; transmitting the ID message signal from the first, wireless, reduced-pressure dressing 112 to the remote base unit 114; receiving the ID message signal at the remote base unit 114; determining if the ID message signal represents a dressing on an approved list; and activating the reduced-pressure source 116 to provide reduced pressure to the distribution manifold 118 if the ID message signal represents a dressing that is on the approved list (or otherwise acceptable) or indicating an error if the ID message signal represents a dressing that is not on the approved list.

Referring now primarily to FIGURE 4, another illustrative embodiment of a first, wireless, reduced-pressure dressing 112 is presented. The first, wireless, reduced-pressure dressing 112 is analogous to the first, wireless, reduced-pressure dressing 112 of FIGURES 1-3 in most respects. A sealing member 120, or film, covers the first RFID antenna 122, first processor 124, and first sensor 126. In this embodiment, a reduced-pressure interface (not shown but analogous to reduced-pressure interface 132 of FIG. 1) may be coupled over an aperture 121 in the sealing member 120 to allow fluid communication, but initially a membrane 160 prevents or hinders flow through the aperture 121.

The membrane 160 may have an occlusive state and a non-occlusive state. The membrane 160 may be in the occlusive state when the membrane 160 is an integral layer and may be non-occlusive when one or more apertures are created in the membrane 160 that allow fluid flow through the membrane. When initially over aperture 121 in the occlusive state, flow through the aperture 121 is prevented (or substantially hindered), but when the membrane 160 is changed to the non-occlusive state, flow through the membrane 160 and, thus, through the aperture 121 may occur. The membrane 160 may move from the occlusive state to the non-occlusive state in response to an activating event, e.g., the application heat, light, ultrasound, a chemical, or other activating agent. For example, in one illustrative embodiment, a dissolution element 162 is disposed proximate to the membrane 160 and is adapted to (or operable to) change the membrane 160 from an occlusive state to a non-occlusive state. The dissolution element 162 may be activated by the first processor 124 to produce the activating event. In other embodiments, the dissolution element 162 may be activated by the second processor 148 or an external stimulus.

The dissolution element 162 may be, for example and not by way of limitation, an electrically resistive heating element. Power may be supplied from the first processor 124 to the dissolution element 162 to sufficiently activate the electrically resistive heating element to melt, dissolve or otherwise create an aperture in the membrane 160. In another illustrative embodiment, the dissolution element 162 is an ultrasonic device that creates an aperture in membrane 160 when activated. In another illustrative embodiment, the dissolution element 162 is a chemical distribution device that upon receiving a signal from the first processor 124 releases an agent that causes the membrane 160 to dissolve, at least in part, to create an aperture 121. In still another illustrative embodiment, the dissolution element 162 is a light with a first wavelength and the membrane 160 reacts to the light with the first wavelength and dissolves at least a portion to create an aperture.

The membrane 160 may be made from numerous materials depending on the activation device used as the dissolution element 162. For example, the membrane 160 may comprise a semi-crystalline thermoplastic film, such as LDPE, HDPE, PP, PA, having a thickness in the range 15 - 100 µm. The exact thickness of the membrane 160 depends on the material selected and the level of reduced pressure that the membrane 160 will be required to resist. The temperature required to rupture the membrane 160 should be approaching or at the melting point of the polymer chosen. If heated whilst the membrane 160 is under strain from applied negative pressure, the temperature required to breakdown or dissolve the membrane 160 may be reduced.

The dissolution element 162 may be a component laminated to the membrane 160 during the manufacture of the membrane 160 or attached later in production. The dissolution element 162 may be a conductive component that is molded into the membrane 160 or added to the membrane 160 so that the membrane 160 itself is conductive. The conductive material may be metallic or one of the other materials that are commonly used to provide conductivity to polymers. Although the temperature required to melt some of the membrane or film materials mentioned may be greater than 100° C, the dissolution element 162 is separated from the patient sufficiently to avoid injury or other potential complications. Moreover, the membrane 160 may be extremely thin such that very little energy is required to form an aperture through the membrane 160.

Referring now primarily to FIGURES 1-4, if the first, wireless, reduced-pressure dressing 112 includes the membrane 160 and dissolution element 162, an initial activation may be carried out by the remote base unit 114 and first processor 124. As an illustrative example, the remote base unit 114 may transmit an activation signal from the remote base unit 114 to the first, wireless, reduced-pressure dressing 112. Upon receiving the activation signal, the first processor 124 activates the dissolution element 162 to change the membrane 160 to the non-occlusive state such as by creating an aperture over the aperture 121. Reduced pressure from the reduced-pressure source 116 may then be delivered through the reduced-pressure interface 132 and the aperture 121 to the distribution manifold 118. If a user is attempting to use a remote base unit that is not designed for use with the first, wireless, reduced-pressure dressing 112, the remote base unit will not respond to the activation signal and will not change the membrane 160 from the occlusive state to the non-occlusive state. Accordingly, the first, wireless, reduced-pressure dressing 112 would be unable to establish fluid flow through the membrane 160 to the distribution manifold 118.

According to one illustrative embodiment, in operation, the first, wireless, reduced-pressure dressing 112 is disposed proximate to the tissue site 102. The distribution manifold 118 is placed adjacent to the tissue site 102. The sealing member 120 is releaseably attached to the epidermis 108 with the attachment device 128. The reduced-pressure delivery conduit 134 is fluidly coupled between the reduced-pressure interface 132 and the reduced-pressure source 116. The remote base unit 114 may be activated by the user with the control panel 158.

The remote base unit 114 may initially transmit an activation signal to the first, wireless, reduced-pressure dressing 112 to cause the membrane 160 to change from an occlusive state to a non-occlusive state as previously described. The remote base unit 114 may then transmit an ID inquiry signal to identify the dressing type. The first, wireless, reduced-pressure dressing 112 receives the ID inquiry signal and, in response, transmits an ID message signal indicative of the dressing type. The second processor 148 may receive the ID message signal and look up the ID message signal or otherwise determine if the dressing represented by the ID message signal is acceptable. If the dressing is acceptable, the second processor 148 may cause a control signal to be sent, e.g., by coupling 140, to the reduced-pressure source 116 to activate the reduced-pressure source 116 and begin treatment with reduced pressure. In another embodiment, the dressing type may be validated before an activation signal is sent.

The remote base unit 114 may be configured to transmit a pressure inquiry signal to the first, wireless, reduced-pressure dressing 112. In response, the first, wireless, reduced-pressure dressing 112 ascertains the pressure with the fist sensor 126 and transmits a pressure message signal to the remote base unit 114. Based on the pressure message signal, the remote base unit 114 determines if the pressure is greater than a first target pressure (on an absolute scale), and if so, continues operation of the reduced-pressure source 116. But if the pressure message signal indicates that the pressure is less than the first target pressure (on absolute scale), the second processor 148 may send a control signal stopping the reduced-pressure source 116 from delivering reduced pressure. Operation of the reduced-pressure source 116 may be by manipulation of a valve or power to a vacuum pump or other like techniques. The remote base unit 114 may interrogate the first, wireless, reduced-pressure dressing 112 from time to time to monitor the pressure. If the pressure becomes greater than the first target pressure (on an absolute scale), the reduced-pressure source 116 will again be activated by a control signal. Thus, a feedback loop may be utilized.

If a patient has a plurality of tissue sites 102 requiring treatment, a plurality of wireless, reduced-pressure dressings may be used. For example, the first, wireless, reduced-pressure dressing 112 may be placed at the first tissue site 102 and a second, wireless, reduced-pressure dressing (not shown, but analogous to the reduced-pressure dressing 112) may be placed at a second tissue site. Because the signals include unique identification information, the remote base unit 114 may communicate with both the first, wireless, reduced-pressure dressing 112 and the second, wireless, reduced-pressure dressing. This arrangement allows monitoring and control of reduced pressure at each of the plurality of tissue sites. Multiple remote base units 114 may also be used. For example, each first processor 124 may include dynamic RAM that includes a register that is configured when paired to a particular remote base unit 114. Thus, the first, wireless, reduced-pressure dressing 112 is able to distinguish which remote base unit 114 has been assigned. The first, wireless, reduced-pressure dressing 112 may include a reset button that transmits a reset signal to the first processor 124 to allow the dressing and base association to be changed.

Referring now primarily to FIGURE 5, another illustrative embodiment of a system 100 for treating at least one tissue site 102 with reduced pressure is presented. The system 100 includes a first, wireless, reduced-pressure dressing 112. The system 100 is analogous in most respects to the system 100 of FIGURE 1, except that a reduced-pressure source 116 is a micro-pump 117, e.g., a piezoelectric pump, and is adjacent to the distribution manifold 118. In addition, an absorbent layer 119 is also explicitly included. As used throughout this document in connection with the micro-pump 117, "adjacent" means next to, in the vicinity of, and also includes in.

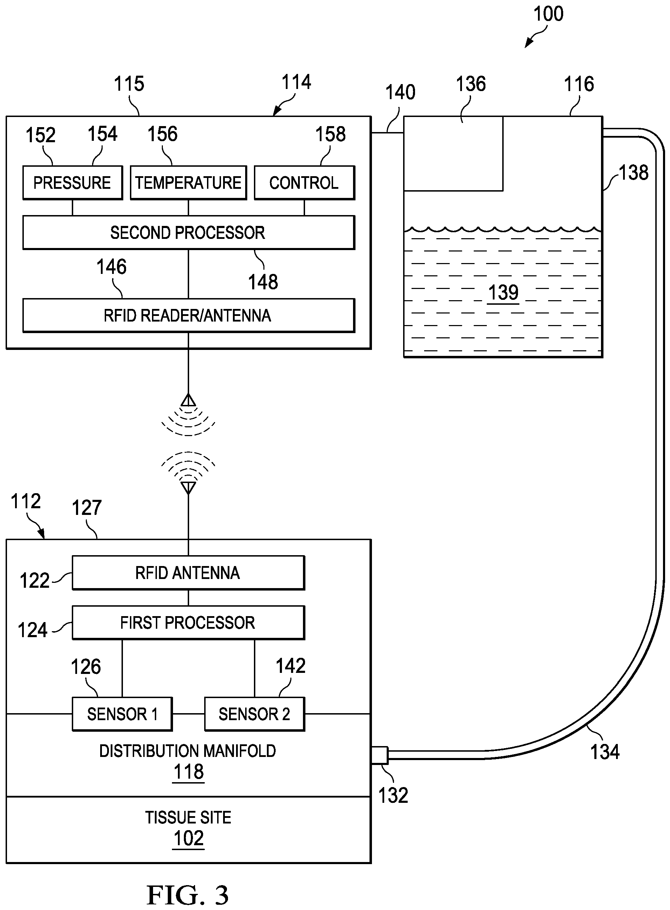

A dedicated battery 166 may be coupled to the micro-pump 117 to provide power to the micro-pump 117. A vent line 168 may be used to allow the micro-pump 117 to vent or exhaust outside of the first, wireless, reduced-pressure dressing 112. In this embodiment, a first processor (analogous to first processor 124 of FIGURE 3) that is associated with a WISP or RFID device 170, may be instructed by the remote base unit 114 to deliver a control signal to the micro-pump 117 when necessary. In another illustrative embodiment, the first processor may receive a pressure message signal indicative of the pressure at the distribution manifold 118, compare the pressure to a target pressure, and in response deliver a control signal to the micro-pump 117 to control the micro-pump 117 as needed. In another illustrative embodiment, the device 170 may harvest power to not only energize the first processor and first sensor, but also the micro-pump 117. It should be noted that a capacitor (not explicitly shown) may be included to help build and retain a charge for powering devices in the first, wireless, reduced-pressure dressing 112.

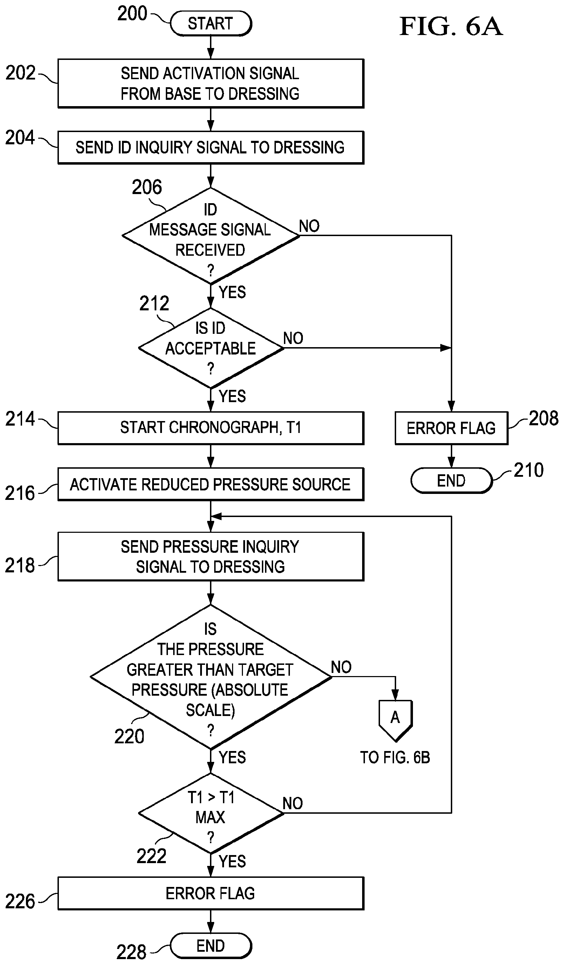

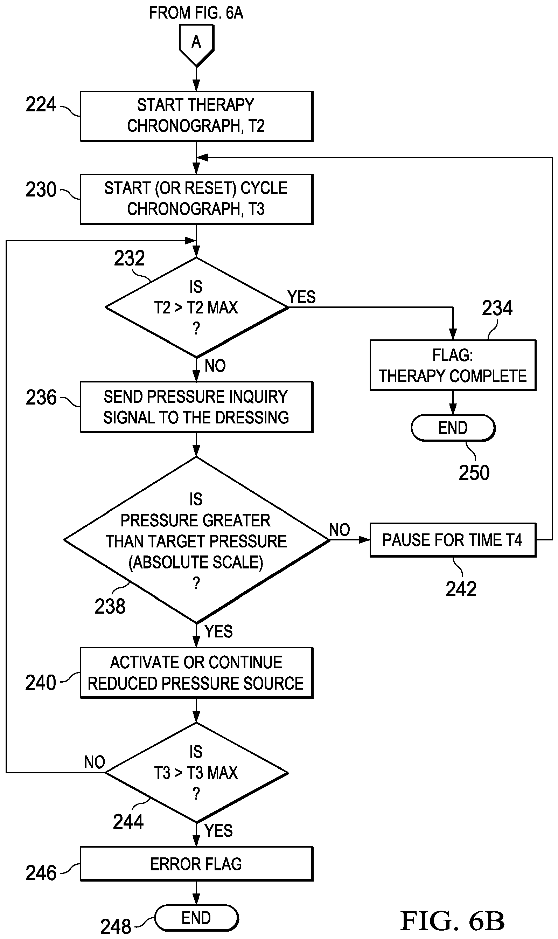

Referring now to FIGURES 3, 4, and 6, and primarily to FIGURE 6, a treatment process that may be executed with the system 100 is presented. The process begins at 200. At step 202, the remote base unit 114 transmits an activation signal to the first, wireless, reduced-pressure dressing 112. The first RFID antenna 122 receives the activation signal. The activation signal energizes the first processor 124. The first processor 124 activates the dissolution element 162. The dissolution element 162 changes the membrane 160 from an occlusive state to a non-occlusive state that allows flow. It should be appreciated that the need to change the membrane 160 to a non-occlusive state helps make sure that only appropriate (approved) remote base units 114 are used with the first, wireless, reduced-pressure dressing 112.

At step 204, the remote base unit 114 transmits an ID inquiry signal to the first, wireless, reduced-pressure dressing 112. The first RFID antenna 122 receives the ID inquiry signal. The ID inquiry signal energizes the first processor 124 and provides an identification request to the first processor 124. In response, the first processor 124 transmits an ID message signal from the first RFID antenna 122 to the RFID reader 146 on the remote base unit 114. The remote base unit 114 waits for the ID message signal at interrogatory 206.

At interrogatory 206, the second processor 148 waits a predetermined time for the ID message signal. If the ID message signal is not received during the predetermined time, the process proceeds to step 208 where an error flag is posted and the process ends at 210. The error flag may include posting a message for display at control panel 158 or sounding an alarm or providing other notification. If the ID message signal is received, the process proceeds to interrogatory 212.

At interrogatory 212, inquiry is made as to whether the ID message signal represents a dressing that is acceptable or approved. To answer the inquiry, the second processor 148 compares the dressing represented by the ID message signal with a list of acceptable or approved dressings. If the dressing represented by the ID message signal is on the list of acceptable or approved dressings, the process continues to step 214 and if not the process proceeds to step 208 and an error flag is posted and the process ends at 210.

At step 214, a first chronograph, T1, is initiated to keep a running cycle time. The process then proceeds to step 216. At step 216, the second processor 148 provides a control signal via coupling 140 to the reduced-pressure source 116 to activate the reduced-pressure source 116. Once the reduced-pressure source 116 is activated, reduced pressure flows to the first, wireless, reduced-pressure dressing 112.

At step 218, the remote base unit 114 transmits a pressure inquiry signal to the first, wireless, reduced-pressure dressing 112. The first RFID antenna 122 receives the pressure inquiry signal. The pressure inquiry signal energizes the first processor 124 and first sensor 126. In response, the first processor 124 transmits a pressure message signal to the remote base unit 114. The RFID reader 146 receives and delivers the pressure message signal to the second processor 148. At interrogatory 220, the second processor 148 then compares the pressure message signal, which indicates the pressure substantially at the distribution manifold 118 or other desired location, with a target pressure. If the pressure message signal indicates a pressure that is greater than the target pressure on an absolute scale, then more reduced pressure is needed and the delivery of reduced pressure continues. The process proceeds to interrogatory 222. On the other hand, if the pressure message signal indicates a pressure that is less than the target pressure on an absolute scale, then the process proceeds to step 224.

At decision step 222, the second processor 148 compares the elapsed time of the first chronograph, T1, with a maximum time, T1max. If the maximum time has been exceeded, the process continues to step 226. At step 226, the second processor 148 posts an error flag and the process ends at 228. The error flag may include sounding an alarm or displaying a message on the control panel 158 or otherwise notifying the user of a problem. If the maximum time has not been exceeded, the process returns to step 218. The loop (218, 220, 222, 218, ...) continues until the maximum time is exceeded or a suitable reduced pressure is reached.

Once the pressure is suitable, the process proceeds to step 224 where the second processor 148 initiates a therapy chronograph that keeps an elapsed time, T2, for therapy. Next, at step 230, the second processor 148 initiates a cycle chronograph, T3. The process then proceeds to interrogatory 232, wherein the elapsed therapy time, T2, is compared to a maximum allowed value. If the elapsed therapy time, T2, is greater than the maximum allowed value, then a flag indicating that therapy is complete is given at step 234 and the process ends 250. Otherwise, the process continues to step 236.

At step 236, the remote base unit 114 transmits a pressure inquiry signal to the first, wireless, reduced-pressure dressing 112. The first RFID antenna 122 receives the pressure inquiry signal. The pressure inquiry signal is then delivered to the first processor 124. The pressure inquiry signal energizes the first processor 124 and the first sensor 126. In response, the first processor 124 develops a pressure message signal. The first processor 124 and first RFID antenna 122 transmit the pressure message signal to the remote base unit 114. The pressure message signal is received and delivered to the second processor 148. At interrogatory 238, the pressure at the distribution manifold 118 (or other desired location) as represented by the pressure message signal is compared to a target value on an absolute scale. If the pressure is greater than the target value, reduced pressure needs to be applied (or continued), and the second processor 148 transmits (or continues) a control signal to the reduced-pressure source 116 at step 240. If the pressure is less than the target value, the process pauses at step 242 and continues to monitor pressure by returning to step 230.

At interrogatory 244, the elapsed cycle time, T3, is compared to a maximum value, T3max. If the elapsed cycle time T3 is greater than the maximum value T3max, the system 100 is apparently unable to adequately lower the pressure. Such a condition may occur because of a leak. Accordingly, an error flag or alarm is initiated at step 246 and the process ends 248. If the elapsed cycle time, T3, is less than the maximum value, T3max, the cycle continues with the process going to interrogatory 232.

The process presented in FIGURES 6A-6B is one illustrative embodiment. Many embodiments are possible. Other process flows may include analogous instructions for receiving temperature message signals from the second sensor 142 or other data depending on the sensor type. In another illustrative embodiment, the activation step may follow the steps required to confirm an approved dressing.

Since the pressure measurements are taken at the first, wireless, reduced-pressure dressing 112 and wirelessly transmitted to the remote base unit 114, the reduced-pressure delivery conduit 134 may be relatively smaller as a pressure sensing lumen is not required as part of the reduced-pressure delivery conduit 134. In other words, pressure is measured and the pressure signal is transmitted to the remote base unit 114 without transmission through a wire and without requiring sampling pressure to be communicated through the reduced-pressure delivery conduit 134.

In another illustrative embodiment, the first, wireless, reduced-pressure dressing may be a distribution manifold enclosed by a drape that has been fenestrated or otherwise allows flow. This illustrative embodiment is suitable for use in a body cavity that is closed, such as placing one in an abdominal cavity and monitoring pressure therein. In such an embodiment, the pressure sensor or other sensors may be located anywhere in or on the dressing and are operable to communicate with a remote base outside of the body cavity. In one embodiment, the pressure sensor may be located at peripheral edge of the dressing that is used in an abdominal cavity. The peripheral edge is disposed near the patient's paracolic gutter to monitor pressure and fluid removal.

The RFID antenna and first processor may be adapted to provide an identification message signal for other purposes than those previously presented. For example, in addition to utilization of the identification message signal to confirm proper pairing of a dressing and base, the identification message signal may be used for inventory purposes. A scanner with a RFID reader may be used to scan a wireless, reduced-pressure dressing and receive the identification message signal.

In another illustrative embodiment, a release pouch is provided that contains a fluid. The release pouch may be formed as part of a wireless, reduced-pressure dressing. The release pouch may be formed, for example, by an additional membrane attached on a patient-facing side of the sealing member. A dissolution element analogous to dissolution element 162 may be associated with the release pouch. The dissolution element allows the first processor 124 to selectively dissolve or open a portion of the release pouch to release any contents of the pouch. Thus, the release pouch may be used to retain medicines or other substances and a wireless signal may be sent to the wireless, reduced-pressure dressing causing release of the medicine. For example, if a sensor detects bacterial colonization, a signal may be sent opening the pouch and releasing an anti-biotic, or if high blood content is detected, a signal may be sent releasing a coagulent.

In some embodiments of the wireless, reduced-pressure dressing, the RFID antenna may be remote from the first processor, but electrically coupled to the first processor. For example, a larger RFID antenna may be used on the patient near the tissue site to be treated, and a coupling wire may extend from the RFID antenna to the first processor or first sensor adjacent to or on the distribution manifold.

According to an illustrative embodiment, a method for treating at least one tissue site with reduced pressure includes disposing a first, wireless, reduced-pressure dressing proximate to a first tissue site, wherein the first, wireless, reduced-pressure dressing comprises: a distribution manifold for placing adjacent to the tissue site and for providing reduced pressure to the tissue site, a sealing member covering at least a portion of the distribution manifold, a first processor, a RFID antenna coupled to the first processor, and a first sensor coupled to the first processor, wherein the first sensor comprises a pressure sensor. The method further includes providing a remote base unit comprising a RFID reader; providing reduced pressure to the distribution manifold; transmitting a first pressure inquiry signal from the RFID reader to the first, wireless, reduced-pressure dressing; and receiving a first pressure message signal from the first, wireless, reduced-pressure dressing in response to the first pressure inquiry signal. The first pressure message signal is wirelessly transmitted by the RFID antenna to the remote base unit without requiring any pressure sensing lumens in the reduced-pressure delivery conduit. The method may further include determining if the first pressure message signal is greater (on an absolute pressure scale) than a target pressure, and if greater than the target pressure, then activating the reduced-pressure source, and if less (on an absolute pressure scale) than the target pressure, then deactivating the reduced-pressure source.

The method of the preceding paragraph may further include disposing a second, wireless, reduced-pressure dressing adjacent to a second tissue site; transmitting a second pressure inquiry signal from the RFID reader to the second, wireless, reduced-pressure dressing; and receiving a second pressure message signal from the second, wireless, reduced-pressure dressing in response to the second pressure inquiry signal. The method may also include using a second sensor coupled to the first processor, wherein the second sensor comprises a temperature sensor.

According to another illustrative embodiment, a system for treating at least one tissue site with reduced pressure includes a first, wireless, reduced-pressure dressing for disposing proximate to the tissue site. The first, wireless, reduced-pressure dressing includes a distribution manifold, a sealing member covering at least a portion of the distribution manifold, a RFID antenna, a first processor coupled to the RFID antenna, and a first sensor coupled to the first processor, a remote base unit comprising a RFID reader and a second processor coupled to the RFID reader. The system also includes a reduced-pressure source fluidly coupled to the distribution manifold.

With respect to the system of the preceding paragraph, the first sensor may be a pressure sensor. The first, wireless, reduced-pressure dressing may further include a second sensor selected from the group: temperature sensor, pH sensor, a humidity sensor, a volatile organic compound sensor, a blood sensor, and a growth factor sensor. The reduced-pressure source may be coupled to the remote base unit and the remote base unit may be adapted to supply a pressure control signal to the reduced-pressure source. In one embodiment, the reduced-pressure source and the remote base unit are an integrated treatment unit. In another aspect, the second processor is adapted to receive an ID signal and to determine if the ID signal represents an approved dressing. The reduced-pressure source may be a micropump adjacent to the distribution manifold. The first, wireless, reduced-pressure dressing may function without a battery. In some embodiments, the first, wireless, reduced-pressure dressing is adjacent to a first tissue site, and further includes a second, wireless, reduced-pressure dressing adjacent to a second tissue site.

According to another illustrative embodiment, a wireless, reduced-pressure dressing for treating a tissue site with reduced pressure includes a distribution manifold for placing adjacent to the tissue site and for providing reduced pressure to the tissue site; a sealing member covering at least a portion of the distribution manifold; a first processor; a RFID antenna coupled to the first processor; and a first sensor coupled to the first processor, wherein the first sensor comprises a pressure sensor. The wireless, reduced-pressure dressing may further include a second sensor coupled to the first processor, wherein the second sensor is selected from the group: temperature sensor, pH sensor, a humidity sensor, a volatile organic compound sensor, a blood sensor, and a growth factor sensor.

According to another illustrative embodiment, a method for treating at least one tissue site with reduced pressure includes the steps of: disposing a first, wireless, reduced-pressure dressing proximate to a first tissue site. The first, wireless, reduced-pressure dressing includes a distribution manifold for placing adjacent to the tissue site and for providing reduced pressure to the tissue site, a sealing member covering at least a portion of the distribution manifold, a first processor, a RFID antenna coupled to the first processor, a first sensor coupled to the first processor, wherein the first sensor comprises a pressure sensor. The method further includes providing a remote base unit comprising a RFID reader. The remote base unit comprises a second processor coupled to the RFID reader. The method also includes fluidly coupling a reduced-pressure source to the distribution manifold; transmitting an ID inquiry signal from the remote base unit to the first, wireless, reduced-pressure dressing; receiving the ID inquiry signal at the first, wireless, reduced-pressure dressing and producing an ID message signal; transmitting the ID message signal from the first, wireless, reduced-pressure dressing to the remote base unit; receiving the ID message signal at the remote base unit; determining if the ID message signal represents a dressing that is on an approved list; and activating the reduced-pressure source to provide reduced pressure to the distribution manifold if the ID message signal represents a dressing that is on the approved list or indicating an error if the ID message signal represents a dressing that is not on the approved list.

In another embodiment, the method of the preceding paragraph may further involve with respect to the first, wireless, reduced-pressure dressing the following: a membrane covering a portion of the distribution manifold, wherein the membrane is initially occlusive, a dissolution element proximate to the membrane and adapted to change the membrane from an occlusive state to a non-occlusive state, and wherein the dissolution element is coupled to the first processor. The method further includes transmitting an activation signal from the remote base unit to the first, wireless, reduced-pressure dressing, whereupon the first processor activates the dissolution element to change the membrane to the non-occlusive state, and providing reduced pressure from the reduced-pressure source to the distribution manifold.

According to another illustrative embodiment, a system for treating at least one tissue site with reduced pressure includes: a first, wireless, reduced-pressure dressing for disposing proximate to the tissue site. The first, wireless, reduced-pressure dressing comprises: a distribution manifold, a sealing member covering at least a portion of the distribution manifold, a reduced-pressure interface for providing reduced pressure to the distribution manifold, a first RFID antenna, a first processor coupled to the first RFID antenna, a first sensor coupled to the first processor, a membrane, wherein the membrane is initially occlusive and wherein the membrane covers at least a portion of the reduced-pressure interface and is adapted to prevent fluid flow when in the occlusive state, a dissolution element proximate to the membrane that is operable, when activated, to change the membrane from an occlusive state to a non-occlusive state, a remote base unit comprising a RFID reader and a second processor; and a reduced-pressure source fluidly coupled to the distribution manifold. The second processor is configured to transmit an activation signal from the RFID reader to the first, wireless, reduced-pressure dressing. In response to the activation signal, the first processor is configured to deliver power to the dissolution element to change the membrane from the occlusive state to the non-occlusive state. The second processor is configured to transmit an ID inquiry signal with the RFID reader to the first, wireless, reduced-pressure dressing. In response to the ID inquiry signal, the first processor is configured to transmit with the first RFID antenna an ID message signal. The second processor is adapted to receive the ID message signal via the RFID reader and to determine if the ID message signal represents a dressing that is on a list of acceptable dressings.

According to another illustrative embodiment, a system for treating a tissue site with reduced pressure includes a wireless, reduced-pressure dressing that includes a distribution manifold, a sealing member covering the distribution manifold, a reduced-pressure interface, and a WISP device associated with the wireless, reduced-pressure dressing. The system further includes a remote base unit comprising a RFID reader for communicating with the WISP device and a reduced-pressure source fluidly coupled to the wireless, reduced-pressure dressing.

According to another illustrative embodiment, a method for treating at least one tissue site with reduced pressure includes disposing a first, wireless, reduced-pressure dressing proximate to a first tissue site. The first, wireless, reduced-pressure dressing includes a distribution manifold for placing adjacent to the tissue site and for providing reduced pressure to the tissue site, a sealing member covering at least a portion of the distribution manifold, a reduced-pressure interface, a membrane, wherein the membrane is initially occlusive and wherein the membrane covers at least a portion of the reduced-pressure interface and is adapted to prevent fluid flow when in the occlusive state, a first processor, a RFID antenna coupled to the first processor, a first sensor coupled to the first processor, wherein the first sensor comprises a pressure sensor, and a dissolution element proximate to the membrane and operable, when activated, to change the membrane from an occlusive state to a non-occlusive state. The dissolution element is coupled to the first processor. The method further includes providing a remote base unit comprising a RFID reader, wherein the remote base unit comprises a second processor; providing a reduced-pressure source fluidly coupled to the reduced-pressure interface; transmitting an activation signal from the remote base unit to the first, wireless, reduced-pressure dressing, whereupon the first processor activates the dissolution element to change the membrane to the non-occlusive state, and providing reduced pressure from the reduced-pressure source to the reduced-pressure interface whereby the non-occlusive state allows fluid flow to the distribution manifold.

According to another illustrative embodiment, a system for treating at least one tissue site includes a first, wireless, reduced-pressure dressing for disposing proximate to the tissue site. The first, wireless, reduced-pressure dressing includes a sealing member, a RFID antenna, a first processor coupled to the RFID antenna, and a first sensor coupled to the first processor; and a remote base unit comprising a RFID reader and a second processor coupled to the RFID reader. The first sensor may be a pressure sensor. The first, wireless, reduced-pressure dressing may further comprise a second sensor selected from the group: temperature sensor, pH sensor, a humidity sensor, a volatile organic compound sensor, a blood sensor, and a growth factor sensor. The reduced-pressure source may be coupled to the remote base unit and the remote base unit may be adapted to supply a pressure control signal to the reduced-pressure source. The reduced-pressure source and the remote base unit may comprise an integrated treatment unit. The second processor may be adapted to receive an ID signal and to determine if the ID signal represents an approved dressing. The first, wireless, reduced-pressure dressing may function without an included a battery. The first, wireless, reduced-pressure dressing may be adjacent to a first tissue site, and further comprises a second, wireless, reduced-pressure dressing adjacent to a second tissue site.

According to another embodiment, a wireless, reduced-pressure dressing for use on a tissue site includes a sealing member for covering at least a portion of the tissue site; a first processor; a RFID antenna coupled to the first processor; and a first sensor coupled to the first processor. The first sensor may be a pressure sensor. The wireless, reduced-pressure dressing may further include a second sensor coupled to the first processor, wherein the second sensor is selected from the group: temperature sensor, pH sensor, a humidity sensor, a volatile organic compound sensor, a blood sensor, and a growth factor sensor. The wireless, reduced-pressure dressing may further include a distribution manifold. The wireless, reduced-pressure dressing may further include a reduced-pressure source fluidly coupled to the reduced-pressure dressing.

Generally, systems, methods, and dressings are presented herein that involve using a RFID device in a dressing to provide pressure data or other data to a remote base unit. The dressing is an interactive dressing. The reduced pressure in the dressing may be monitored and used to control delivery of reduced pressure. In one instance, the pressure data at each dressing allows a plurality of tissue sites to be monitored and used with as single remote base unit. In another instance, devices and methods for assuring a proper pairing of reduced-pressure dressings and remote base units are also presented. Other systems, methods, and dressings are presented.

Although the present invention and its advantages have been disclosed in the context of certain illustrative, non-limiting embodiments, it should be understood that various changes, substitutions, permutations, and alterations can be made without departing from the scope of the invention as defined by the appended claims. It will be appreciated that any feature that is described in connection to any one embodiment may also be applicable to any other embodiment.

It will be understood that the benefits and advantages described above may relate to one embodiment or may relate to several embodiments. It will further be understood that reference to "an" item refers to one or more of those items.

The steps of the methods described herein may be carried out in any suitable order, or simultaneously where appropriate.

Where appropriate, aspects of any of the embodiments described above may be combined with aspects of any of the other embodiments described to form further examples having comparable or different properties and addressing the same or different problems.

It will be understood that the above description of preferred embodiments is given by way of example only and that various modifications may be made by those skilled in the art. The above specification, examples and data provide a complete description of the structure and use of exemplary embodiments of the invention. Although various embodiments of the invention have been described above with a certain degree of particularity, or with reference to one or more individual embodiments, those skilled in the art could make numerous alterations to the disclosed embodiments without departing from the scope of the claims.

## Claims

1. A method for controlling a reduced-pressure treatment system, the method comprising:
disposing a first, wireless, reduced-pressure dressing (112) proximate to a first tissue site (102), wherein the first, wireless, reduced-pressure dressing (112) comprises:
a distribution manifold (118) for placing adjacent to the tissue site (102) and for providing reduced pressure to the tissue site,
a sealing member (120) covering at least a portion of the distribution manifold (118),
a first processor (124),
a RFID antenna (122) coupled to the first processor (124), and
a first sensor (126) coupled to the first processor (124), wherein the first sensor (126) comprises a pressure sensor;
providing a remote base unit (114) comprising a RFID reader (146);
transmitting a first pressure inquiry signal from the RFID reader (146) to the first, wireless, reduced-pressure dressing (112);
harvesting energy from the first pressure inquiry signal to energize the
first processor (124) and the first sensor (126);
receiving a first pressure message signal from the first, wireless, reduced-pressure dressing (112) in response to the first pressure inquiry signal; and
wherein the first pressure message signal is wirelessly transmitted by the RFID antenna (122) to the remote base unit (114) without requiring any pressure sensing lumens in a reduced-pressure delivery conduit.

2. The method of claim 1, further comprising determining if the first pressure message signal is greater (on an absolute pressure scale) than a target pressure, and if greater than the target pressure, then activating a reduced-pressure source (116), and if less (on an absolute pressure scale) than the target pressure, then deactivating the reduced-pressure source (116).

3. The method of claim 1 or claim 2, wherein the method further comprises:
disposing a second, wireless, reduced-pressure dressing adjacent to a second tissue site;
transmitting a second pressure inquiry signal from the RFID reader to the second, wireless, reduced-pressure dressing; and
receiving a second pressure message signal from the second, wireless, reduced-pressure dressing in response to the second pressure inquiry signal.

4. The method of any of the preceding claims, wherein the first, wireless, reduced-pressure dressing (112) further comprises a second sensor (142) coupled to the first processor (124), wherein the second sensor (142) comprises a temperature sensor.

5. The method of claim 1, further comprising:
fluidly coupling a reduced-pressure source (116) to the distribution manifold (118);
transmitting an ID inquiry signal from the remote base unit (114) to the first, wireless, reduced-pressure dressing (112);
receiving the ID inquiry signal at the first, wireless, reduced-pressure dressing (112) and producing an ID message signal;
transmitting the ID message signal from the first, wireless, reduced-pressure dressing (112) to the remote base unit (114);
receiving the ID message signal at the remote base unit (114);
determining if the ID message signal represents a dressing that is on an
approved list;
activating the reduced-pressure source (116) to provide reduced pressure to the distribution manifold if the ID message signal represents a dressing that is on the approved list or indicating an error if the ID message signal represents a dressing that is not on the approved list.

6. The method of claim 5,
wherein the first, wireless, reduced-pressure dressing (112) further comprises:
a membrane (160) covering a portion of the distribution manifold, wherein the membrane is initially occlusive,
a dissolution element (162) proximate to the membrane (160) and adapted to change the membrane from an occlusive state to a non-occlusive state, and
wherein the dissolution element (162) is coupled to the first processor (124); and
wherein the method further comprises:
transmitting an activation signal from the remote base unit (114) to the first, wireless, reduced-pressure dressing (112), whereupon the first processor (124) activates the dissolution element (162) to change the membrane (160) to the non-occlusive state, and
providing reduced pressure from the reduced-pressure source (116) to the distribution manifold (118).

7. A system for treating at least one tissue site with reduced pressure, the system comprising:
a first, wireless, reduced-pressure dressing for disposing proximate to the tissue site, wherein the first, wireless, reduced-pressure dressing comprises:
a distribution manifold,
a sealing member covering at least a portion of the distribution manifold,
a RFID antenna,
a first processor coupled to the RFID antenna,
a first sensor coupled to the first processor, and
power harvesting circuitry for harvesting power from radio signals emitted from an RFID reader to energize the first processor.

8. The system of claim 7, further comprising a reduced-pressure source fluidly coupled to the distribution manifold.

9. The system of claim 7 or 8, wherein the first sensor is a pressure sensor and the first, wireless, reduced-pressure dressing further comprises a second sensor selected from the group: temperature sensor, pH sensor, a humidity sensor, a volatile organic compound sensor, a blood sensor, and a growth factor sensor.

10. The system of any one of claims 7-9, further comprising an RFID reader adapted to transmit a radio signal to the RFID antenna.

11. The system of any one of claims 7-10, wherein the second processor is adapted to receive an ID signal and to determine if the ID signal represents an approved dressing.

12. The system of any one of claims 8-11, wherein the reduced-pressure source comprises a micropump adjacent to the distribution manifold.

13. The system of claim 7 wherein the first, wireless, reduced-pressure dressing (112) further comprises:
a membrane (160) covering a portion of the distribution manifold, wherein the membrane is initially occlusive;
a dissolution element (162) proximate to the membrane (160) and adapted to change the membrane from an occlusive state to a non-occlusive state, and wherein the dissolution element (162) is coupled to the first processor (124).

## Patentansprüche

1. Verfahren zum Steuern eines Unterdruck-Behandlungssystems, wobei das Verfahren umfasst:
Anordnen eines ersten, drahtlosen, Unterdruckverbands (112) nahe einer ersten Gewebestelle (102), wobei der erste, drahtlose, Unterdruckverband (112) umfasst:
einen Verteiler (118) zum Anordnen benachbart zu der Gewebestelle (102) und zum Bereitstellen von Unterdruck an die Gewebestelle,
ein Dichtungselement (120), das mindestens einen Abschnitt des Verteilers (118) bedeckt,
einen ersten Prozessor (124),
eine RFID-Antenne (122), die mit dem ersten Prozessor (124) verbunden ist, und einen ersten Sensor (126), der mit dem ersten Prozessor (124) verbunden ist, wobei der erste Sensor (126) einen Drucksensor umfasst;
Bereitstellen einer Fernbasiseinheit (114), die einen RFID-Leser (146) umfasst;
Senden eines ersten Druckabfragesignals von dem RFID-Leser (146) an den ersten, drahtlosen, Unterdruckverband (112);
Gewinnen von Energie aus dem ersten Druckabfragesignal, um den ersten Prozessor (124) und den ersten Sensor (126) mit Energie zu versorgen;
Empfangen eines ersten Druckmitteilungssignals von dem ersten, drahtlosen, Unterdruckverband (112) als Reaktion auf das erste Druckabfragesignal; und
wobei das erste Druckmitteilungssignal durch die RFID-Antenne (122) drahtlos an die Fernbasiseinheit (114) gesendet wird, ohne Druckerfassungslumina in einer Unterdruckzuführleitung zu erfordern.

2. Verfahren nach Anspruch 1, das ferner ein Bestimmen, ob das erste Druckmitteilungssignal (auf einer Absolutdruckskala) größer ist als ein Solldruck, und, wenn größer als der Solldruck, dann ein Aktivieren einer Unterdruckquelle (116) und, wenn (auf einer Absolutdruckskala) kleiner als der Solldruck, dann ein Deaktivieren der Unterdruckquelle (116) umfasst.

3. Verfahren nach Anspruch 1 oder 2, wobei das Verfahren ferner umfasst:
Anordnen eines zweiten, drahtlosen, Unterdruckverbands benachbart zu einer zweiten Gewebestelle;
Senden eines zweiten Druckabfragesignals von dem RFID-Leser an den zweiten, drahtlosen, Unterdruckverband; und
Empfangen eines zweiten Druckmitteilungssignals von dem zweiten, drahtlosen, Unterdruckverband als Reaktion auf das zweite Druckabfragesignal.

4. Verfahren nach einem der vorhergehenden Ansprüche, wobei der erste, drahtlose, Unterdruckverband (112) ferner einen zweiten Sensor (142) umfasst, der mit dem ersten Prozessor (124) verbunden ist, wobei der zweite Sensor (142) einen Temperatursensor umfasst.

5. Verfahren nach Anspruch 1, ferner umfassend:
strömungstechnisches Verbinden einer Unterdruckquelle (116) mit dem Verteiler (118);
Senden eines ID-Abfragesignals von der Fernbasiseinheit (114) an den ersten, drahtlosen, Unterdruckverband (112);
Empfangen des ID-Abfragesignals an dem ersten, drahtlosen, Unterdruckverband (112) und Erzeugen eines ID-Mitteilungssignals;
Senden des ID-Mitteilungssignals von dem ersten, drahtlosen, Unterdruckverband (112) an die Fernbasiseinheit (114);
Empfangen des ID-Mitteilungssignals an der Fernbasiseinheit (114);
Bestimmen, ob das ID-Mitteilungssignal für einen Verband steht, der sich auf einer genehmigten Liste befindet;
Aktivieren der Unterdruckquelle (116), um Unterdruck an den Verteiler bereitzustellen, wenn das ID-Mitteilungssignal für einen Verband steht, der sich auf der genehmigten Liste befindet, oder Anzeigen eines Fehlers, wenn das ID-Mitteilungssignal für einen Verband steht, der sich nicht auf der genehmigten Liste befindet.

6. Verfahren nach Anspruch 5,
wobei der erste, drahtlose, Unterdruckverband (112) ferner umfasst:
eine Membran (160), die einen Abschnitt des Verteilers bedeckt, wobei die Membran anfänglich okklusiv ist,
ein Auflösungselement (162) nahe der Membran (160) und dazu ausgelegt, die Membran von einem okklusiven Zustand in einen nicht okklusiven Zustand zu bringen, und
wobei das Auflösungselement (162) mit dem ersten Prozessor (124) verbunden ist; und
wobei das Verfahren ferner umfasst:
Senden eines Aktivierungssignals von der Fernbasiseinheit (114) an den ersten, drahtlosen, Unterdruckverband (112),
woraufhin der erste Prozessor (124) das Auflösungselement (162) aktiviert, um die Membran (160) in den nicht okklusiven Zustand zu bringen, und
Bereitstellen von Unterdruck von der Unterdruckquelle (116) an den Verteiler (118).

7. System zum Behandeln mindestens einer Gewebestelle mit Unterdruck, wobei das System umfasst:
einen ersten, drahtlosen, Unterdruckverband zum Anordnen nahe der Gewebestelle, wobei der erste, drahtlose, Unterdruckverband umfasst:
einen Verteiler,
ein Dichtungselement, das mindestens einen Abschnitt des Verteilers bedeckt,
eine RFID-Antenne,
einen ersten Prozessor, der mit der RFID-Antenne verbunden ist,
einen ersten Sensor, der mit dem ersten Prozessor verbunden ist, und
eine Energiegewinnungsschaltung zum Gewinnen von Energie aus Funksignalen,
die von einem RFID-Leser ausgestrahlt werden, um den ersten Prozessor mit Energie zu versorgen.

8. System nach Anspruch 7, das ferner eine Unterdruckquelle umfasst, die strömungstechnisch mit dem Verteiler verbunden ist.

9. System nach Anspruch 7 oder 8, wobei der erste Sensor ein Drucksensor ist und der erste, drahtlose, Unterdruckverband ferner einen zweiten Sensor umfasst, der ausgewählt ist aus der Gruppe: Temperatursensor, pH-Wert-Sensor, ein Feuchtigkeitssensor, ein Sensor für flüchtige organische Verbindungen, ein Blutsensor und ein Wachstumsfaktorsensor.

10. System nach einem der Ansprüche 7 - 9, das ferner einen RFID-Leser umfasst, der dazu ausgelegt ist, ein Funksignal an die RFID-Antenne zu senden.

11. System nach einem der Ansprüche 7 - 10, wobei der zweite Prozessor dazu ausgelegt ist, ein ID-Signal zu empfangen und zu bestimmen, ob das ID-Signal für einen genehmigten Verband steht.

12. System nach einem der Ansprüche 8 - 11, wobei die Unterdruckquelle eine Mikropumpe umfasst, die dem Verteiler benachbart ist.

13. System nach Anspruch 7, wobei der erste, drahtlose, Unterdruckverband (112) ferner umfasst:
eine Membran (160), die einen Abschnitt des Verteilers bedeckt, wobei die Membran anfänglich okklusiv ist;
ein Auflösungselement (162) nahe der Membran (160) und dazu ausgelegt, die Membran von einem okklusiven Zustand in einen nicht okklusiven Zustand zu bringen, und wobei das Auflösungselement (162) mit dem ersten Prozessor (124) verbunden ist.

## Revendications

1. Procédé pour commander un système de traitement par pression réduite, le procédé comprenant le fait de :
disposer un premier pansement sans fil à pression réduite (112) à proximité d'un premier site tissulaire (102), le premier pansement sans fil à pression réduite (112) comprenant :
un collecteur de distribution (118) destiné à venir se placer en position adjacente au site tissulaire (102) et à procurer une pression réduite au site tissulaire ;
un élément d'étanchéisation (120) recouvrant au moins une portion du collecteur de distribution (118) ;
un premier processeur (124) ;
une antenne RFID (122) couplée au premier processeur (124) ; et
un premier capteur (126) couplé au premier processeur (124), le premier
capteur (126) comprenant un capteur de pression ;
procurer une unité de base à distance (114) comprenant un lecteur RFID (146) ;
transmettre un premier signal de demande de pression à partir du lecteur RFID (146) au premier pansement sans fil à pression réduite (112) ;
récolter de l'énergie à partir du premier signal de demande de pression afin d'exciter le premier processeur (124) et le premier capteur (126) ;
recevoir un premier signal de message de pression à partir du premier pansement sans fil à pression réduite (112) en réponse au premier signal de demande de pression ; et
dans lequel le premier signal de message de pression est transmis sans fil par l'antenne RFID (122) à l'unité de base à distance (114) sans nécessiter une quelconque lumière de détection de pression dans un conduit de distribution de pression réduite.

2. Procédé selon la revendication 1, comprenant en outre le fait de déterminer si le premier signal de message de pression est supérieur (sur une échelle de pression absolue) à une pression cible, et lorsqu'il est supérieur à la pression cible, d'activer une source de pression réduite (116) et, lorsqu'il est inférieur (sur la même échelle de pression absolue) à la pression cible, de désactiver la source de pression réduite (116).

3. Procédé selon la revendication 1 ou 2, dans lequel le procédé comprend en outre le fait de :
disposer un second pansement sans fil à pression réduite en position adjacente à un second site tissulaire ;
transmettre un second signal de demande de pression à partir du lecteur RFID au second pansement sans fil à pression réduite ; et
recevoir un second signal de message de pression à partir du second pansement sans fil à pression réduite en réponse au second signal de demande de pression.

4. Procédé selon l'une quelconque des revendications précédentes, dans lequel le premier pansement sans fil à pression réduite (112) comprend en outre un second capteur (142) couplé au premier processeur (124), le second capteur (142) comprenant un capteur de température.

5. Procédé selon la revendication 1, comprenant en outre le fait de :
coupler par fluide une source de pression réduite (116) au collecteur de distribution (118) ;
transmettre un signal de demande d'identité à partir de l'unité de base à distance (114) au premier pansement sans fil à pression réduite (112) ;
recevoir le signal de demande d'identité au premier pansement sans fil à pression réduite (112) et produire un signal de message d'identité ;
transmettre le signal de message d'identité à partir du premier pansement sans fil à pression réduite (112) à l'unité de base à distance (114) ;
recevoir le signal de message d'identité à l'unité de base à distance (114) ;
déterminer si le signal de message d'identité représente un pansement qui est repris dans une liste approuvée ;
activer la source de pression réduite (116) pour procurer une pression réduite au collecteur de distribution lorsque le signal de message d'identité représente un pansement qui est repris dans la liste approuvée ou indiquer une erreur lorsque le signal de message d'identité représente un pansement qui n'est pas repris dans la liste approuvée.

6. Procédé selon la revendication 5 :
dans lequel le premier pansement sans fil à pression réduite (112) comprend en outre :
une membrane (160) recouvrant une portion du collecteur de distribution, la membrane étant initialement occlusive ;
un élément de dissolution (162) à proximité de la membrane (160) et conçu pour modifier la membrane en le faisant passer d'un état occlusif à un état non conclusif; et dans lequel l'élément de dissolution (162) est couplé au premier processeur (124) ; et
dans lequel le procédé comprend en outre le fait de :
transmettre un signal d'activation à partir de l'unité de base à distance (114) au premier pansement sans fil à pression réduite (112); après quoi, le premier processeur (124) active l'élément de dissolution (162) pour modifier la membrane (160) et la faire passer à l'état non occlusif ; et
procurer une pression réduite à partir de la source de pression réduite (116) au collecteur de distribution (118).

7. Système pour traiter au moins un site tissulaire avec une pression réduite, le système comprenant :
un premier pansement sans fil à pression réduite destiné à venir se disposer à proximité du site tissulaire, le premier pansement sans fil à pression réduite comprenant :
un collecteur de distribution ;
un élément d'étanchéisation recouvrant au moins une portion du collecteur de distribution ;
une antenne RFID ;
un premier processeur couplé à l'antenne RFID ;
un premier capteur couplé au premier processeur ; et
un circuit de récolte d'énergie pour récolter de l'énergie à partir de signaux radio émis par un lecteur RFID afin d'exciter le premier processeur.

8. Système selon la revendication 7, comprenant en outre une source de pression réduite couplée par fluide au collecteur de distribution.

9. Système selon la revendication 7 ou 8, dans lequel le premier capteur est un capteur de pression et le premier pansement sans fil à pression réduite comprend en outre un second capteur choisi parmi le groupe : un capteur de la température, un capteur du pH, un capteur de l'humidité, un capteur des composés organiques volatils, un capteur du sang et un capteur de facteur de croissance.

10. Système selon l'une quelconque des revendications 7 à 9, comprenant en outre un lecteur RFID conçu pour transmettre un signal radio à l'antenne RFID.

11. Système selon l'une quelconque des revendications 7 à 10, dans lequel le second processeur est conçu pour recevoir un signal d'identité et pour déterminer si le signal d'identité représente un pansement approuvé.

12. Système selon l'une quelconque des revendications 8 à 11, dans lequel la source de pression réduite comprend une micropompe en position adjacente au collecteur de distribution.

13. Système selon la revendication 7, dans lequel le premier pansement sans fil à pression réduite (112) comprend en outre :
une membrane (160) recouvrant une portion du collecteur de distribution, la membrane étant initialement occlusive ;
un élément de dissolution (162) à proximité de la membrane (160) et conçu pour modifier la membrane en le faisant passer d'un état occlusif à un état non conclusif; et dans lequel l'élément de dissolution (162) est couplé au premier processeur (124).
